# EUROPEAN PATENT APPLICATION

(11) **EP 2 765 412 A1**
(43) Date of publication of application: **13.08.2014**
(21) Application number: 14153984.1
(22) Date of filing: 05.02.2014
(51) Int. Cl.: G01N 27/327, C12Q 1/00, G01N 33/14

(54) **Biosensor and measuring method employing same**

(30) Priority: 12.02.2013 JP 2013024243
(71) Applicant: Tanita Corporation, Tokyo 174 (JP)
(72) Inventor: Koide, Satoshi, Itabashi-ku Tokyo 174-8630 (JP)
(74) Representative: Grünecker, Kinkeldey, Stockmair & Schwanhäusser

(57) **Abstract**

A biosensor (100) is a biosensor for measuring concentrations of first and second substrates in a sample, including a sensor unit (101) including an insulating base plate (111), an electrode (116) over the insulating base plate (111), and a reaction layer (114) over the electrode (116); a timing unit (103) for measuring time; and a calculating unit (105), in which the reaction layer (114) contains a first enzyme for converting the first substrate into the second substrate, and a second enzyme acting on the second substrate or the converted substance obtainable by further converting the second substrate, and the calculating unit (105) calculates concentrations of the first and the second substrates in the sample, on the basis of a first current value detected in the sensor unit (101) at a first time and a second current value detected in the sensor unit (101) at a second time.

## Description

### TECHNICAL FIELD

The present invention relates to a biosensor and a measuring method employing thereof.

### RELATED ART

Technologies related to biosensors include one described in Japanese Laid-Open Patent Application Publication No. 2010-54379. Japanese Laid-Open Patent Application Publication No. 2010-54379 describes biosensors, which is capable of electrochemically detecting a reaction of a substrate in a sample with an oxidoreductase to determine a substrate concentration, and also describes that this achieves allowing a design of a calibration range to a desired range according to the substrate concentration contained in a sample while accurately measuring the substrate concentration within a desired range.

Also, technologies related to sucrose sensors include those described in Japanese Laid-Open Patent Application Publication No. 2001-174432, Japanese Laid-open patent publication No. H06-88805 (1994) and Japanese Laid-open patent publication No. H09-229895 (1997). Among these, Japanese Laid-open patent publication No. H09-229895 (1997) describes a technology for detecting the total quantity of glucose, fructose and sucrose.

Technologies related to cholesterol sensors include those described in Japanese Re-Publication of WO01/038862, Japanese Patent Domestic Publication No. 2009-520974, Japanese Patent Domestic Publication No. 2009-537020, Japanese Laid-open patent publication No. H10-232219 (1998), Japanese Laid-open patent publication No. H11-5189 (1999), Japanese Laid-open patent publication No. H11-2618 (1999), Japanese Laid-open patent publication No. 2005-83965. Among these, Japanese Patent Domestic Publication No. 2009-520974 describes determining contents of total cholesterol, triglyceride and high density lipoprotein (HDL) cholesterol in a sample by providing first to third electrochemical cells. Also, Japanese Patent Domestic Publication No. 2009-537020 describes a use of a first cell for conducting an HDL cholesterol test and a second cell for conducted a total cholesterol test.

Japanese Laid-open patent publication No. H08-338826 (1996) and Japanese Laid-open patent publication No. H08-336399 (1996) describe a use of a sensor having an arginine sensor and a urea sensor to determine arginine contained in a sample containing arginine and urea.

Also, Japanese Laid-open patent publication No. H11-271258 (1999) describes a technology related to multiple-sensors including individual sensors arranged therein, each of which is dedicated for measuring each of Na⁺, glucose and urea, respectively.

### SUMMARY OF THE INVENTION

However, there are needs to be improved in the conventional technologies described in the above-described Documents, in terms of achieving simple and stable measurements for concentrations of a plurality of substrates contained in a sample. Taking the sucrose sensors described in Japanese Laid-Open Patent Application Publication No. 2001-174432, Japanese Laid-open patent publication No. H06-88805 (1994) and Japanese Laid-open patent publication No. H09-229895 (1997) as examples, it is difficult to easily determine respective concentrations of glucose and sucrose in a sample containing glucose and sucrose by employing these sucrose sensors.

According to one aspect of the present invention, there is provided a biosensor for measuring concentrations of a first and a second substrates in a sample, including a sensor unit including a base plate, an electrode provided over the base plate, and a reaction layer provided over the electrode; a timing unit for measuring time; and a calculating unit, in which the reaction layer contains a first enzyme for converting the first substrate into the second substrate, and a second enzyme acting on the second substrate or the converted substance obtainable by further converting the second substrate, and in which the calculating unit calculates a concentration of the first substrate and a concentration of the second substrate in the sample respectively, on the basis of a first current value detected in the sensor unit at a first time and a second current value detected in the sensor unit at a second time.

According to another aspect of the present invention, there is provided a method of measuring the first and the second substrate concentrations in a sample by employing the aforementioned biosensor according the present invention, including: acquiring the first current value detected at the first time in the sensor unit; acquiring the second current value detected at the second time in the sensor unit; and calculating a concentration of the first substrate and a concentration of the second substrate in the sample respectively, on the basis of the first and the second current values.

In addition to above, any arbitrary combination of each of these constitutions or conversions between the categories of the invention such as a process, a device, a method for utilizing the device and the like may also be within the scope of the present invention.

For example, there is provided a use of the aforementioned biosensor according the present invention for measurement of the first and the second substrate concentrations in the sample.

According to the present invention, the concentrations of a plurality of substrates contained in a sample can be easily and stably measured.

### BRIEF DESCRIPTION OF THE DRAWINGS

The above and other objects, advantages and features of the present invention will be more apparent from the following description of certain preferred embodiments taken in conjunction with the accompanying drawings, in which:
Fig. 1 is a block diagram, schematically illustrating a configuration of a biosensor in an embodiment;
Fig. 2 is a cross-sectional view, illustrating a configuration of a sensor unit in the biosensor in the embodiment;
Fig. 3 is a plan view, illustrating the configuration of the sensor unit in the biosensor in the embodiment;
Fig. 4 is a cross-sectional view, illustrating a configuration of a sensor unit in a biosensor in an embodiment;
Fig. 5 is a perspective view, illustrating a configuration of a sensor unit in a biosensor in an embodiment;
Fig. 6 includes graphs, showing measurement results of current values employing the biosensor in Examples; and
Fig. 7 includes graphs, showing measurement results of current values employing the biosensor in Examples.

### DETAILED DESCRIPTION

The invention will be now described herein with reference to illustrative embodiments. Those skilled in the art will recognize that many alternative embodiments can be accomplished using the teachings of the present invention and that the invention is not limited to the embodiments illustrated for explanatory purposed.

Exemplary implementations according to the present invention will be described in detail as follows in reference to the annexed figures. In all figures, similar numeral is assigned to similar constituent element, and the duplicated descriptions are not presented.

### (First Embodiment)

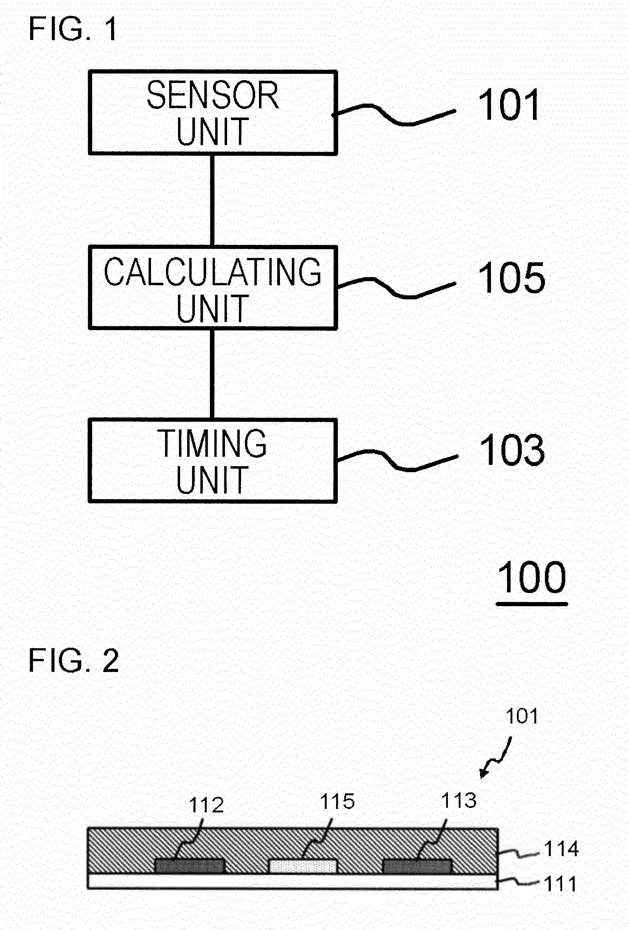

Fig. 1 is a block diagram, which schematically represents a configuration of a biosensor in an embodiment. A biosensor 100 shown in Fig. 1 is a biosensor that is capable of measuring concentrations of a first and a second substrate in a sample. The biosensor 100 includes a sensor unit 101, a timing unit 103, and a calculating unit 105.

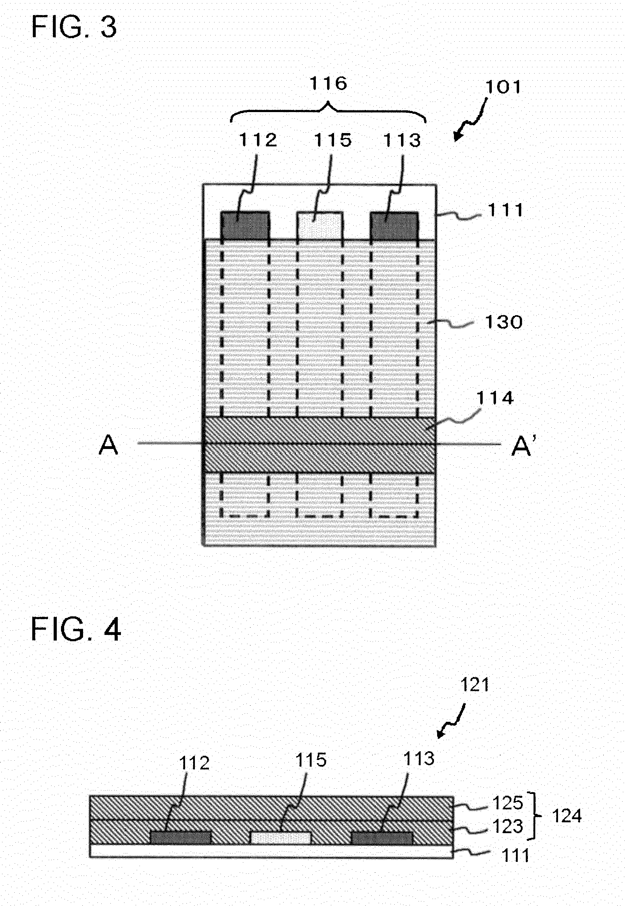

Fig. 2 and Fig. 3 are diagrams, which illustrate a configuration of the sensor unit 101. Fig. 3 is a plan view, illustrating the configuration of the sensor unit 101, and Fig. 2 is a cross-sectional view of Fig. 3 along the line A-A'. The sensor unit 101 includes a base plate (insulating base plate 111), electrodes 116 provided on the insulating base plate 111, and a reaction layer 114 provided over the electrode 116.

Ceramics, plastics, silicon, alumina glass plate or macromolecular materials may be employed for the insulating base plate 111. Preferably, a plate of synthetic resin such as polyethylene terephthalate, polyvinyl chloride and polycarbonate and the like may be employed.

For an example a bipolar electrode or two-electrode system composed of a working electrode 112 and a counter electrode 113 may be adopted for the electrode 116. The working electrode 112 and the counter electrode 113 are formed of electroconductive materials. The electroconductive materials are typically carbon, or the metallic materials such as palladium, silver, platinum, gold, copper, nickel, alloys thereof and the like.

Alternatively, tripolar electrode or three-electrode system composed of a working electrode 112, a counter electrode 113 and a reference electrode 115 as shown in Fig. 2 and Fig. 3 may also be adopted as the electrode 116. The use of the three-electrode system allows stably obtaining a response current to stabilize the measurement accuracy. The reference electrode 115 is also composed of an electroconductive material, similarly as in the working electrode 112 and the counter electrode 113.

The identical material may be employed for all of the electrodes, or different material may alternatively be employed for respective electrodes. The electrode may be formed of a single type of electroconductive material, or may be formed of a combination of two or more types of electroconductive materials. This allows acquiring a stable response current, so that the accuracy is further stabilized.

The working electrode 112, the counter electrode 113 and the reference electrode 115 may be produced by, for example, conducting screen printing, sputter process, or vapor deposition process to provide a coating over the insulating base plate 111.

Further, the electrode 116 is provided with an insulating region having a surface, which is covered with an insulating film 130, a measurement region, which is covered with a reaction layer 114, and an applying region for applying an electric voltage. In the applying region, a surface of the electrode 116 is exposed.

Also, the electrode 116 has a single working electrode 112, and the reaction layer 114 is provided on the working electrode 112.

The reaction layer 114 contains a first enzyme for converting a first substrate into a second substrate, and a second enzyme acting on the second substrate or on the converted substance obtainable by further converting the second substrate. In Fig. 2 and Fig. 3, the reaction layer 114, is provided so as to be in contact with the electrode 116.

The first enzyme may be, for example, a hydrolase. Also, the second enzyme may be, for example, an oxidoreductase. Specific examples and combinations of the first and the second enzymes and the first and the second substrates will be discussed later.

Alternatively, the reaction layer 114 may further contain an enzyme except for the first and the second enzymes. For example, it may be configured that the reaction layer 114 further contains a third enzyme for converting the second substrate into the converted substance thereof and the second enzyme acts on the above-described converted substance of the second substrate.

The contents of the first and the second enzymes in the reaction layer 114 are not particularly limited, and an appropriate amount may be selected as required.

For example, when invertase is employed as the first enzyme, it is preferable that the content thereof is 0.001 to 10 unit/µL. Here, "1 unit" in this case is an amount of hydrolase, which is required for achieving hydrolysis of 1 µmol of the substrate in 1 minute.

Also, when glucose oxidase (GOx), which is an oxidoreductase, is employed as the second enzyme, it is preferable that the content thereof is 0.1 to 50 unit/µL. Here, "1 unit" in this case is an amount of oxidoreductase, which is required for achieving oxidation of 1 µmol of the substrate in 1 minute.

While the thickness of the reaction layer 114 is not in particular limited, it is preferable to be 0.01 to 10 µm, in view of reducing the amount of the sample employing in the measurement as possible, as in the case that the sample is, for example, blood or the like, and more preferably 0.025 to 10 µm, and further preferably 0.05 to 5 µm.

The timing unit 103 is configured to measures the time.

The calculating unit 105 acquires the time measured by the timing unit 103. The calculating unit 105 also acquires the current value, which is measured by the sensor unit 101. The calculating unit 105, in turn, conducts a calculation on the basis of data acquired from the timing unit 103 and the sensor unit 101.

Specifically, the calculating unit 105 calculates a concentration of the first substrate and a concentration of the second substrate in the sample respectively, on the basis of a first current value (I1) detected in the sensor unit 101 at a first time (T1) and a second current value (12) detected in the sensor unit 101 at a second time (T2).

More specifically, the calculating unit 105 calculates the concentration of the second substrate in the sample on the basis of I1, and also calculates the concentration of the first substrate in the sample on the basis of a difference between I2 and I1 (I2-I1).

Typical measurement sample includes: biological fluids such as blood, urine, saliva, sudor, lacrima and the like; foods such as fruit juice, soup and the like;
cell culture mediums;
fermented samples (food or non-food); and
industrial products and food additives such as polyamino acid, disaccharide to polysaccharide and the like. A solid food may also be an object for the measurement, if an object for the measurement can be easily extracted with an aqueous solution or the like.

Next, specific examples of the measuring method employing the biosensor 100 will be described.

The measuring method of the present embodiment includes, for example, the following process steps:
acquiring a current value I1 detected at the sensor unit 101 at time T1;
acquiring a current value 12 detected at the sensor unit 101 at time T2; and
calculating a concentration of the first substrate and a concentration of the second substrate in a sample respectively, on the basis of I1 and I2.

The following description will be made in reference to an example of a biosensor, which is capable of being used for the measurement of concentrations of glucose and sucrose in a sample containing glucose and sucrose. In the embodiment, the first substrate is sucrose, and the second substrate is glucose.

Scheme 1 represents formula illustrating reaction example when the first substrate is sucrose and the second substrate is glucose. In addition to above, "FAD" in the following scheme is an abbreviation of flavin adenine dinucleotide.

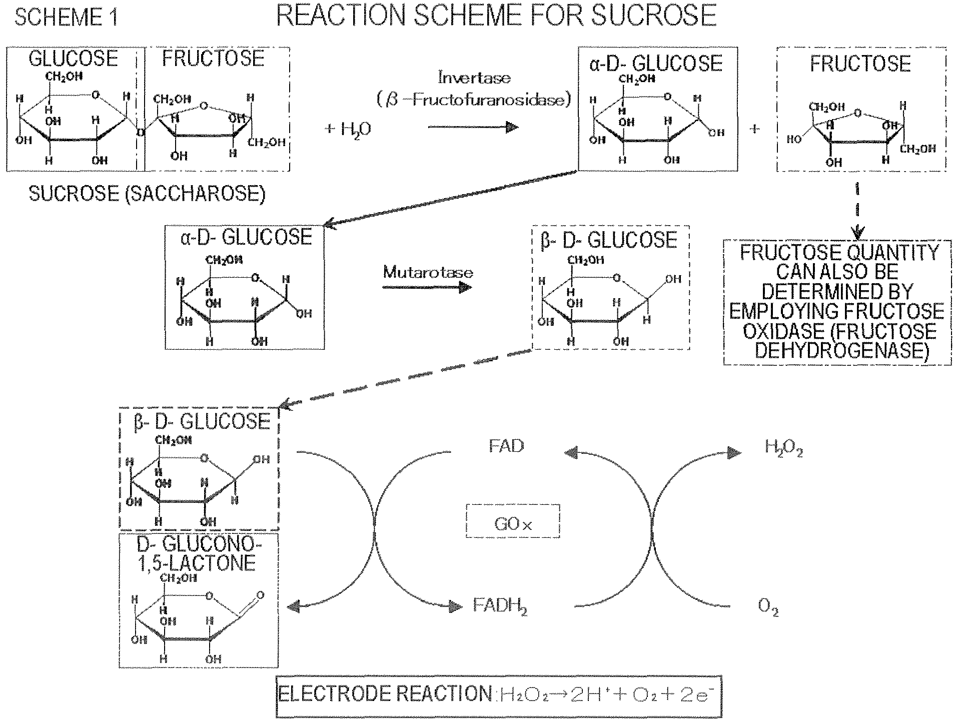

In Scheme 1, sucrose (saccharose), which is the first substrate, is decomposed by an action of invertase (β-fructofuranosidase), which is the first enzyme, into glucose (α-D glucose) and fructose.

Further, α-D-glucose is converted into β-D-glucose by an action of mutarotase, which is the third enzyme. Action of glucose oxidase (referred to as "GOx" in Scheme 1), which is the second enzyme, with β-D-glucose, which is the converted substance of α-D-glucose, induces the following reaction.

More specifically, a DC voltage is applied to the working electrode 112 by a DC power supply (not shown) to cause an oxidation of glucose by the action of glucose oxidase to cause a change to gluconolactone and a generation of hydrogen peroxide, as in the following reaction formula (I).

glucose (C₆H₁₂O₆) + O₂ -> gluconolactone (C₆H₁₀O₆) + H₂O₂ (I)

GOx is reduced by the catalytic reaction of GOx to cause an oxidation of glucose in the measurement sample into gluconic acid, as shown in the reaction formula (I). An electric voltage is applied to the working electrode 112 in the sensor unit 101 to oxidize GOx (reduced form) into GOx (oxidized form). The change of the current occurred by the reaction indicated in the reaction formula (I) is detected to allow measuring the glucose content in the sample.

Further, hydrogen peroxide created from oxygen dissolved in the sample is oxidized on the electrode, and the oxidation current value at that time is also measured to provide the glucose concentration. At this time, hydrogen peroxide generated in reaction formula (I) is oxidized by the electrode reaction on the working electrode 112 to create electron, as in the following reaction formula (II).

H₂O₂ -> 2H⁺ + O₂ + 2e⁻ (II)

In the reaction formula (II), an electric current flows from the working electrode 112 to the counter electrode 113 by electron generated by the hydrogen peroxide reaction. The, this is detected with a circuit for detecting electric current (not shown), and the detected current is processed by the calculating unit 105 to determine the concentration of glucose in the sample. At this time, the potential at the counter electrode 113 is suitably controlled so as to constantly maintain the potential between the working electrode 112 and the reference electrode 115.

Meanwhile, when a plurality of substrate concentrations are measured by using a conventional sensor such as a biosensor for measuring a specific substrate such as sucrose, the method for indirectly measuring the substrate is employed, in which the substrate is once decomposed by an enzyme, and the material created by the decomposition (glucose or fructose) is measured. However, when the material created by the decomposition is also originally contained in the sample, the current value detected in this case is in the form of the added value, and therefore this method cannot achieve the measurement of the substrate alone. For example, in the case of glucose, it is the added value of glucose or fructose, which is created by the enzyme reaction and glucose or fructose originally contained in the sample. Thus, the material, for example, glucose and fructose in the above-described example, which is originally contained in the sample, must be separately measured. However, two types of materials cannot be easily measured at the same time by using the conventional biosensor, and thus it is necessary to employ separate biosensors for measuring respective substrate concentrations.

On the contrary, the measurements by using the same sensor unit 101 in the biosensor 100 in the present embodiment are conducted at two different time T1 and time T2 to obtain the concentration of two or more types of substrates on the basis of information of current measurement values obtained at T1 and T2, so that a plurality of measurements for the concentrations of the substrates can be stably carried out in simple and easy manner.

For example, when the measurement of the sample containing glucose is conducted by employing the biosensor 100, the current value that depends upon the glucose concentration is obtained at the time T1 after the start of the measurement (T0). Further, when the sample containing sucrose is measured, the current value that depends upon the sucrose concentration is obtained at the time T2 after the further passage of time from the time T1. Further, the current value of the glucose component created by sucrose is added for the sample containing glucose and sucrose, unlike the case of a response current value of glucose.

As mentioned above, the calculating unit 105 calculates the glucose concentration by employing the current value I1 is measured at time T1 as the current value derived from glucose originally contained in the sample. Further, for example, the current value at the time T2 is assumed as the added value of the current value derived from the glucose component created by sucrose and the current value derived from glucose originally contained in the sample, such that the difference from the current value derived from glucose originally contained in the sample is calculated to obtain the calculated sucrose concentration in the sample.

For example, the calculating unit 105 maintains the correspondence relation between the sucrose concentration and the response current value as a first calibration curve data, in relation to the measurement value of the standard sample containing sucrose. Further, for example, the calculating unit 105 also maintains the correspondence relation between the glucose concentration and the response current value as a second calibration curve data, in relation to the measurement value of the standard sample containing glucose. In such case, calculating unit 105 can calculate the glucose concentration by referencing the current value I1 at the time I1 with the second calibration curve data, and can also calculate the sucrose concentration by referencing the difference of the current value (I2-I1) with the first calibration curve data.

Subsequently, an example of a process for producing the biosensor 100 will be described.

First of all, a base plate is prepared, and a known method such as screen printing, sputter process, or vapor deposition process or the like is conducted to form the working electrode 112 over the base plate. Subsequently, the screen printing of the insulating paste is conducted over the base plate to form the insulating film 130, thereby obtaining the insulating base plate 111. The insulating film 130 is formed so as to cover the outer circumference of the working electrode 112. The counter electrode 113 and the reference electrode 115 are provided so as to be in parallel with the working electrode 112.

Thereafter, Reaction layer 114 containing the first and the second enzymes is formed on the insulating base plate 111. More specifically, the first and the second enzymes are prepared by being mixed in the aqueous solvent, and the resultant mixture is applied over the surface of the insulating base plate 111, and the applied coating film is dried. It is preferable that the drying is conducted at a temperature that does not cause an inactivation of the oxidoreductase and that is equal to or lower than the optimal temperature. The biosensor 100 is obtained according to the above-described procedure.

Subsequently, a method for measuring concentrations of a plurality of substrates contained in a sample solution by employing the biosensor 100 will be described.

First, a measurement sample is added to the reaction layer 114. The reaction layer 114 is dissolved by the addition of the measurement sample to promote a reaction between the substrate in the sample and oxidoreductase. In order to proceed the reaction, after the biosensor 100 is left for a predetermined time, a pulse voltage (0 to +0.3 V) is applied to the working electrode 112 against the counter electrode 113 toward the direction to the anode. This generates the response current, so that the response current values at the time T1 and the time T2 are measured. The calculating unit 105 acquires the current values I1 and I2 measured by the sensor unit at the time T1 and the time T2 to calculate the concentrations of the first substrate and the second substrate. The calculating unit 105 refers to a calibration curve data related to the first substrate and the second substrate, respectively, which are previously prepared by using a standard sample containing the substrate at a known concentration, to calculate the concentrations of the respective substrates from the response current values. The calibration curve data may be, for example, data, in which the response current values are related with the first or the second substrate concentration and time.

The use of the biosensor 100 allows acquiring the concentrations of a plurality of substrates contained in a sample easily by employing a single sensor.

When the first and the second substrates are contained in a sample and the first substrate is a type of a material that is capable of being changed into the second substrate, the measured values of the response currents are overlapped due to the duplication of the second substrate in the case of the conventional sensor, and thus it is difficult to measure the concentrations for the second substrate, which is originally contained in the sample and the second substrate, which is newly generated due to the first substrate, respectively, by using a single sensor. The difficulty can be overcome by employing the biosensor 100 in the present embodiment, and the concentrations of the first and the second substrates can be obtained by the single measurement.

Further, the biosensor 100 can also be employed to quantitatively measure the time-dependent changes of, for example, the type of the component, which is capable of changing in the production process or in the storage process. Thus, when the biosensor 100 is employed in the measurement of the food sample, the sample for the clinical practice or the sample for industry, it can also be employed for the quality controls of the foods, medical supplies, industrial products and the like in the manufacturing process or in the storage.

In addition to above, when the biosensor 100 is configured of a glucose sensor, glucose dehydrogenase may be employed as oxidoreductase, in place of glucose oxidase.

Further, in scheme 1, the second substrate may alternatively be fructose. In such case, oxidoreductase for fructose may be employed as the second enzyme in place of oxidoreductase for glucose to measure quantity of fructose at the time T1 and at the time T2, so that the quantities of fructose and sucrose contained in the sample can be calculated, respectively. When the quantity of fructose is measured, typical example of oxidoreductase employed as the second enzyme includes, for example fructose oxidase or fructose dehydrogenase.

Further, the first substrate measured by using the biosensor 100 is not limited to sucrose. The first substrate typically includes, for example, saccharides, and more specifically includes: disaccharides such as sucrose, maltose, trehalose, cellobiose and the like; trisaccharides such as raffinose and the like; polysaccharides such as starch and the like. Typical example of a combination of enzymes in the case for measuring saccharide may include: a use of an enzyme as the first enzyme, which is capable of breaking sugar composed of bound two or more monosaccharides into the unit monosaccharide, and a use of an enzyme as the second enzyme, which is capable of oxidizing monosaccharide (glucose oxidase, galactose oxidase and the like).

Specific examples of reaction schemes in the cases that the first substrate is maltose, lactose, trehalose, and raffinose are shown in schemes 2 to 5.

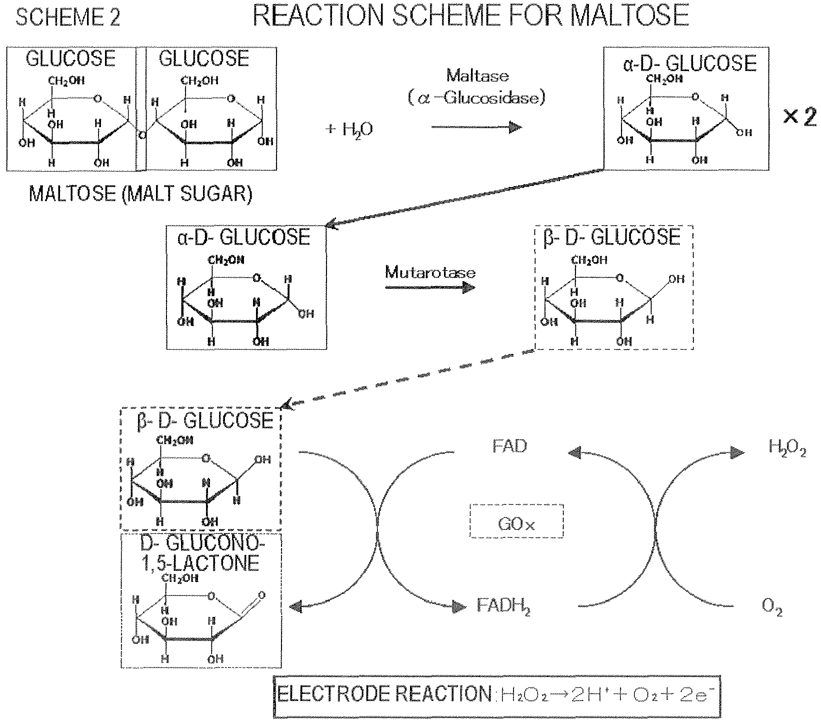

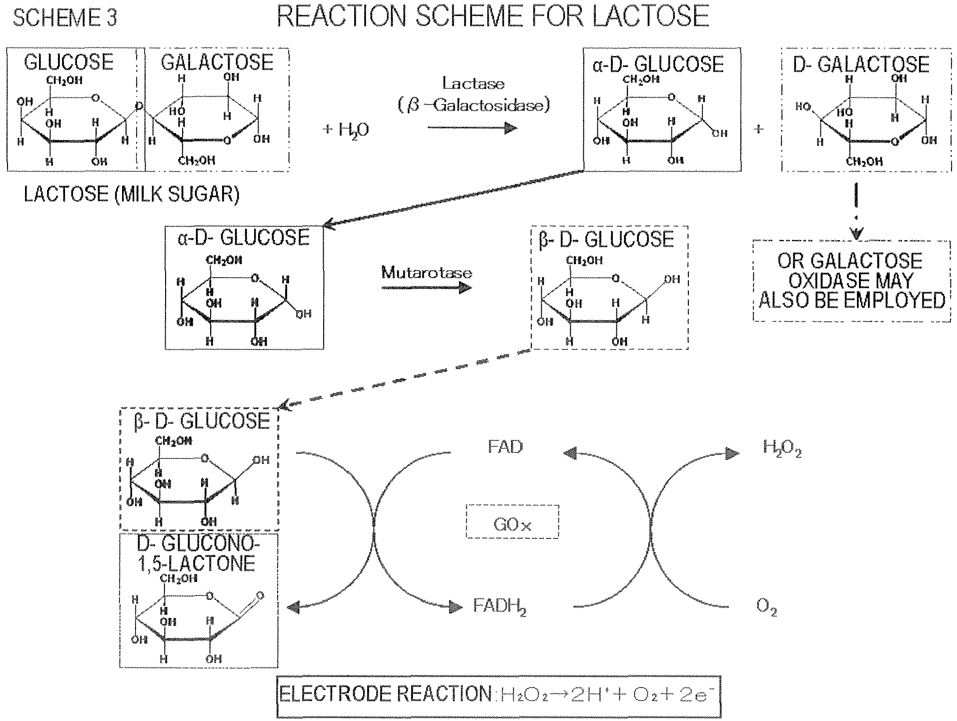

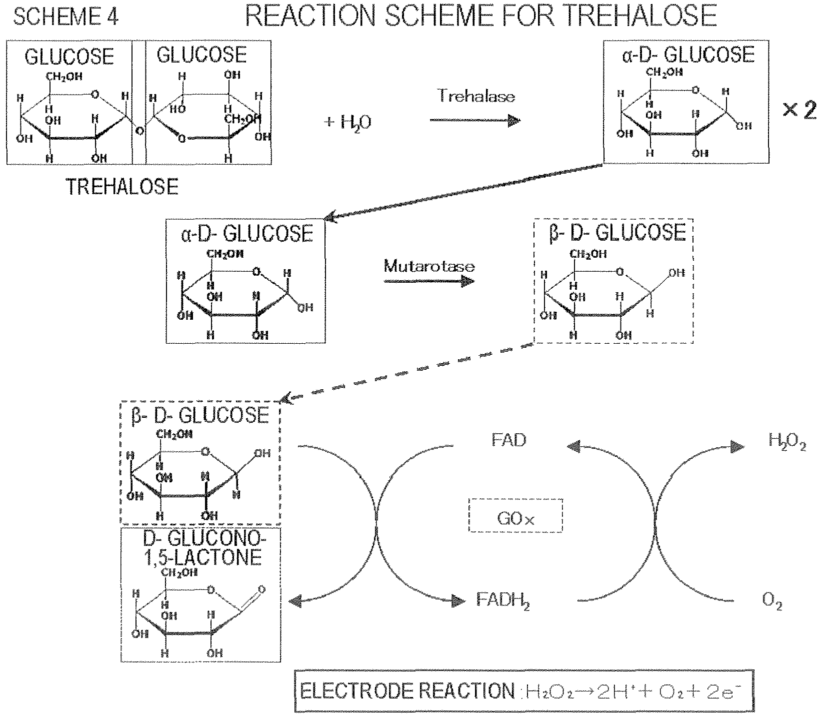

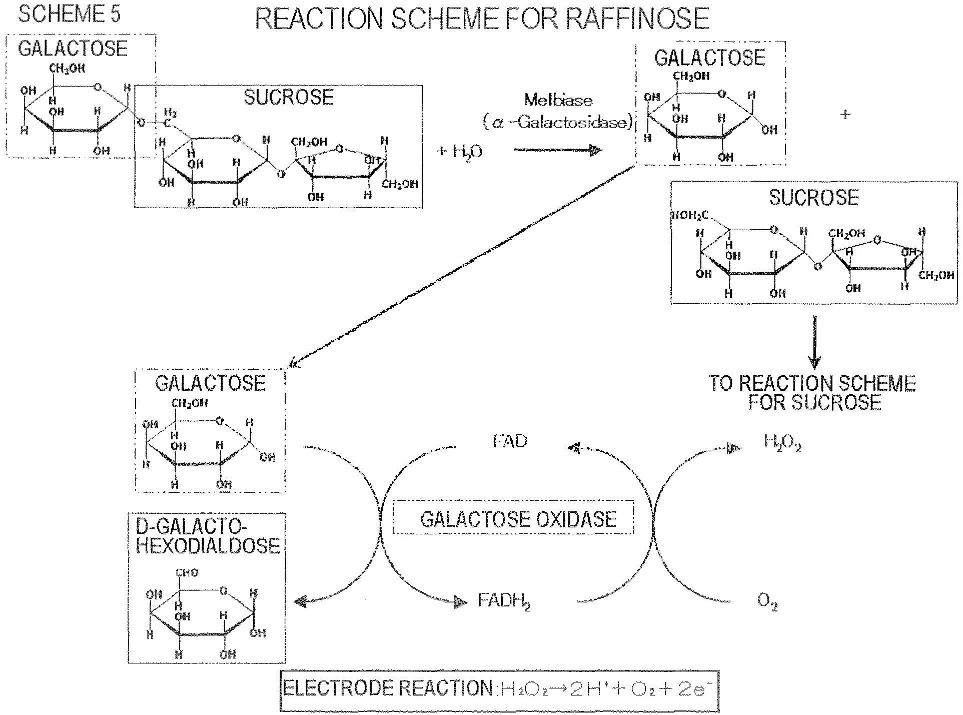

Further, the first substrate also typically includes materials composed of amino acid such as peptide, protein, amino acid polymer and the like. Typical example of a combination of enzymes in the case for measuring peptide-based material may include: a use of an enzyme as the first enzyme, which is capable of breaking peptide bond to decompose into amino acid units; and a use of an enzyme as the second enzyme, which is capable of oxidizing amino acids (glutamate oxidase or the like).

A typical example of a peptide-based material is a reaction scheme of peptide having glutamic acid at the terminal end as shown in scheme 6.

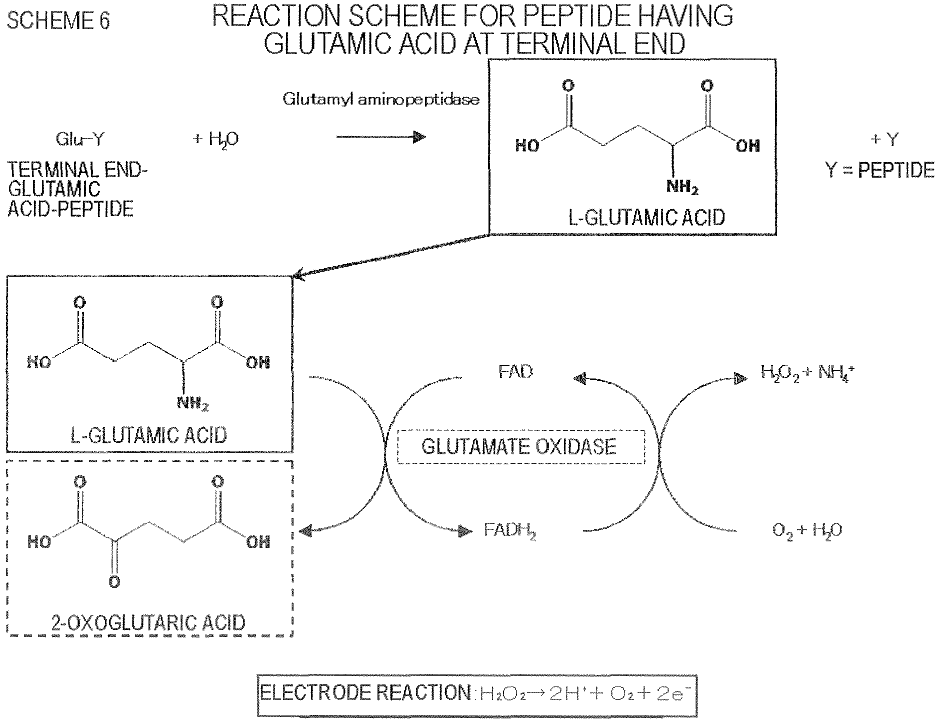

Scheme 6 allows obtaining the concentration of glutamic acid in the peptides contained in a sample containing the peptide having a terminal end of glutamate residue and the concentration of free glutamic acid contained in the sample.

In scheme 6, hydrolysis of glutamate residue at the terminal end of the peptide is caused by the action of glutamyl aminopeptidase serving as the first enzyme to create L-glutamic acid. Glutamate oxidase serving as the second enzyme acts on the created L-glutamic acid to generate an electric current, which depends upon the concentration of L-glutamic acid.

The sensor unit 101 measures the current values I1 and I2 at the time T1 and at the time T2, respectively, and the concentration of glutamic acid in the peptide and the concentration of free glutamic acid are calculated at the calculating unit 105. Then, the calculating unit 105 calculates the concentration of free glutamic acid from the value of I1, and also conducts a predetermined calculation (I2-I1, for example) from the values of I2 and I1 to calculate the concentration of glutamic acid in the peptide. The sensor, which is capable of achieving the quantification of the concentration of glutamic acid, is preferably employed as a sensor for savoriness or umami. Also, the sensor, which is capable of achieving the quantification of the concentration of glutamic acid, may also be employed for the measurement of the time-dependent change of umami material.

Further, the first substrate also typically includes sterol esters such as cholesteryl ester and the like. In such case, an enzyme for decomposing sterol esters such as sterol esterase and the like into sterol and fatty acid may be assigned as the first enzyme, and a reductase for sterol such as cholesterol oxidase and the like may be assigned as the second enzyme.

Reaction scheme of cholesteryl ester is shown in Scheme 7.

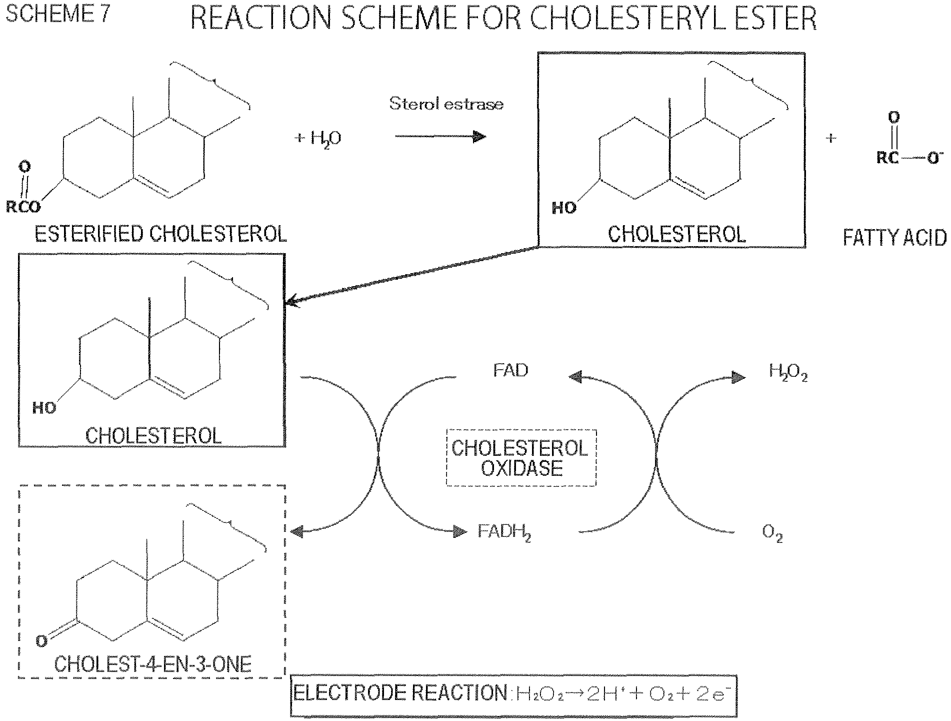

In addition, other examples of the first substrate may include urea, creatinine, cystathionine and the like. Specific examples of reaction schemes of these materials are shown in Schemes 8 to 10.

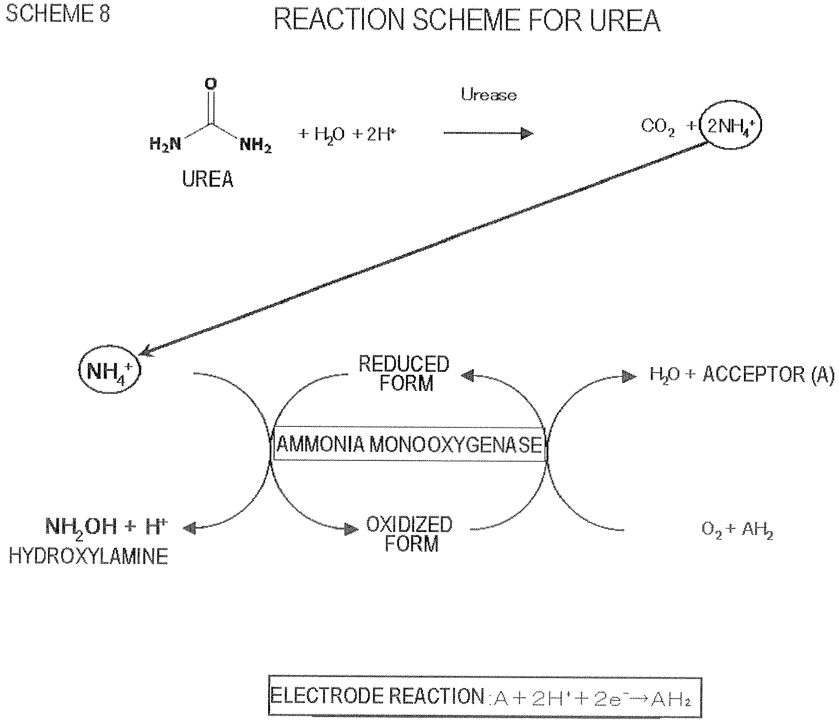

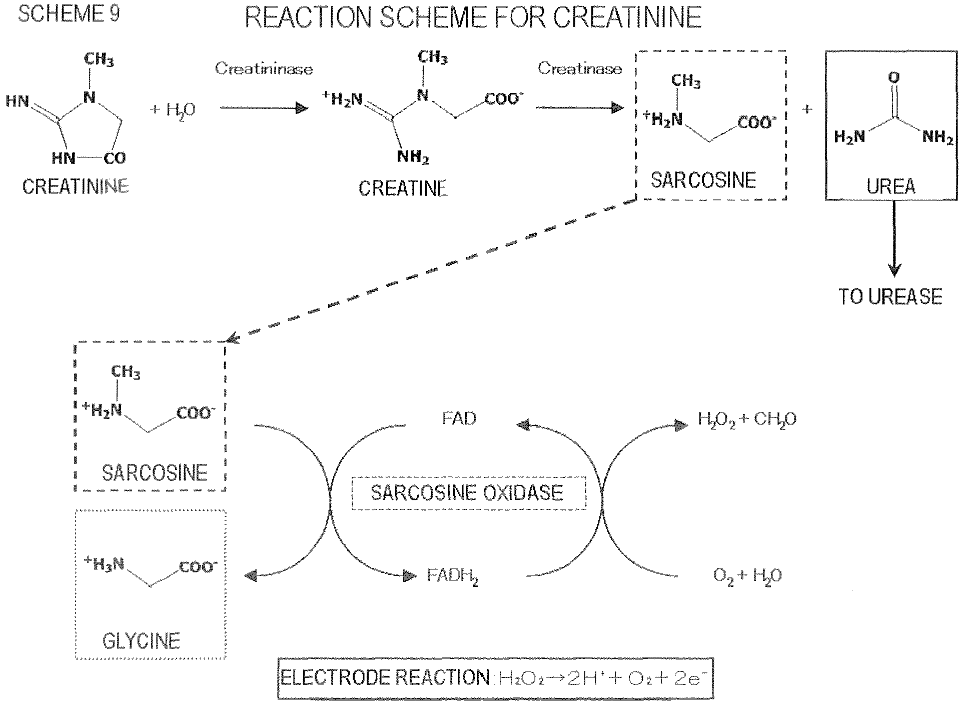

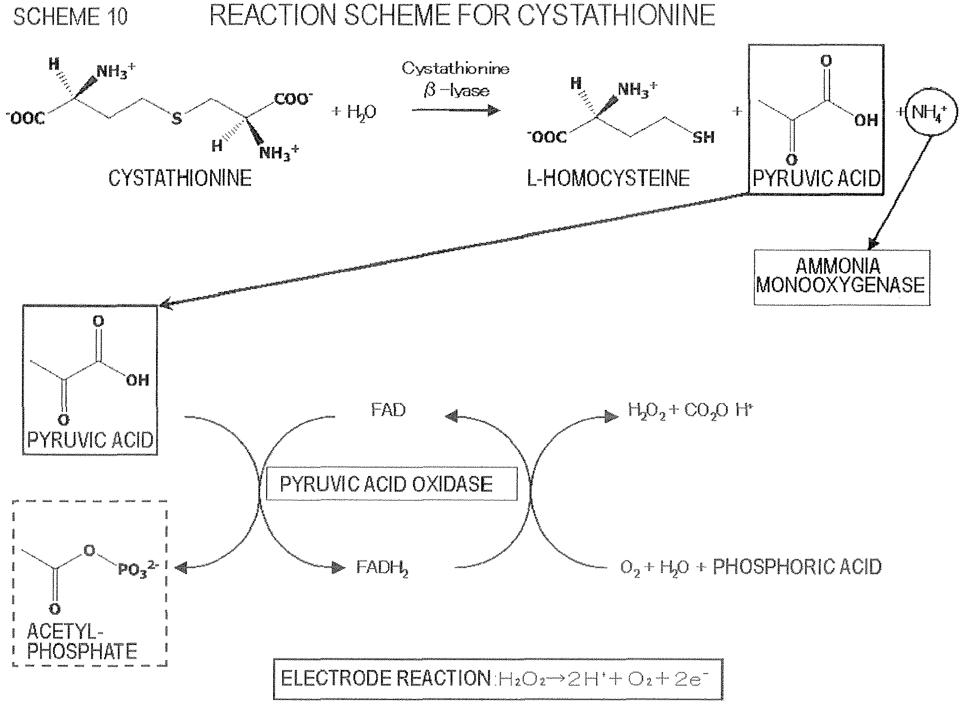

Typical combinations of the respective enzymes and the respective substrates for the biosensor 100 may include, for example, the following combinations.

For example, the first substrate may be one selected from group consisting of sucrose, maltose, lactose and trehalose, and the second substrate may be glucose, and the second enzyme may be glucose oxidase or glucose reductase.

Alternatively, the second substrate may be glutamic acid, and the second enzyme may be glutamate oxidase.

Alternatively, the first substrate may be one selected from group consisting of cholesteryl ester, urea, creatinine, cystathionine and raffinose.

In the descriptions of the following embodiments, the descriptions will be made focusing on features that are different from first embodiment. In addition, suitable combination of the aspects of the respective embodiments may also be employed.

### (Second Embodiment)

The configuration of first embodiment may alternatively be configured that the reaction layer 114 of the sensor unit has a first reaction layer containing a first enzyme and a second reaction layer containing a second enzyme.

Fig. 4 is a cross-sectional view, illustrating a configuration of a sensor unit in the present embodiment. In Fig. 4, a reaction layer 124 of a sensor unit 121 is configured so that a first reaction layer 123, which is in contact with an electrode 116 and a second reaction layer 125, which is in contact with the first reaction layer 123 are stacked. The reaction layer 124 may also be configured so that, for example, the first enzyme is present in the second reaction layer 125 and the second enzyme is present in the first reaction layer 123.

The use of the sensor unit 121 of the present embodiment allows changing the method of immobilizing the enzymes in response to the property of the first and the second enzymes.

### (Third Embodiment)

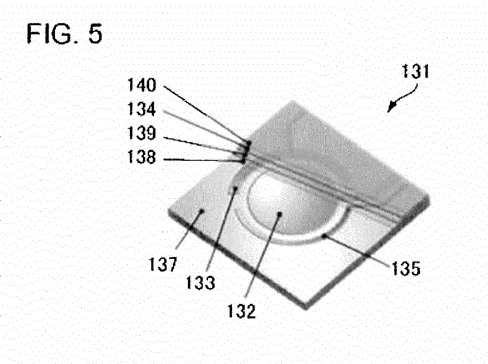

In the above-described embodiments, the sensor unit may alternatively be configured as described below. Fig. 5 is a perspective view, illustrating a configuration of a sensor unit in the present embodiment.

In a sensor unit 131 shown in Fig. 5, a working electrode (platinum electrode) 132, a counter electrode (platinum electrode) 133 and a reference electrode (silver/silver chloride electrode) 135 are provided on a glass base plate 137. The counter electrode 133 and the reference electrode 135 are provided in the different locations along the outer circumference of the working electrode 132 so as to surround the working electrode 132.

In addition, an adhesive layer 138 for covering the electrodes, a selective permeation layer 139, an enzyme layer 134 and a limited permeation layer 140 are provided on the glass base plate 137 in this sequence from the side of the base plate.

The adhesive layer 138 functions as providing improved adhesiveness between a layer disposed over such layer and the glass base plate 137 and the respective electrodes formed under such layer. In addition, such adhesive layer also provides improved wettability of the surface of the glass base plate 137, so that such layer also exhibits an advantageous effect for providing improved uniformity of the thickness of the formed enzyme layer 134. Further, such layer also has selective permeability for ascorbic acid, uric acid and acetaminophen, which may cause interference with the reaction of hydrogen peroxide at the electrode.

Typical example of a material constituting the adhesive layer 138 may include, for example, a silane coupling agent. Typical types of the silane coupling agent may include aminosilane, vinylsilane, epoxysilane and the like, and among these, γ-aminopropyl triethoxysilane, which is a type of aminosilane, is preferable, in view of the adhesiveness and the selective permeability.

The selective permeation layer 139 functions as preventing the permeation of materials other than the materials of the final object for the measurement related to the electrochemical reaction on the electrode surface of the sensor unit 131, and such function is exhibited by the film structure having a stitch structure, which prevents the permeation of molecule having large molecular weight, and further having electrostatic repulsive force, which prevents the penetration of ion. The selective permeation layer 139 is composed of a material selected from, for example, cellulose acetates and ion exchange resins.

The limited permeation layer 140 serves as limiting the diffusion rate of the components to be measured and diminishing the influence of the interfering substance or the obstructing substance, which contributes improvement in the measurement accuracy and expansion of the available range for the measurement. For example, poly dimethylsiloxane or fluoroalcohol ester of polycarboxylic acid may be preferably employed for the limited permeation layer 140. Here, fluoroalcohol ester of polycarboxylic acid is a product by an esterification of some of or the whole carboxylic group of polycarboxylic acid with fluoroalcohol. In addition, fluoroalcohol is a product by a substitution of all or at least one of hydrogen in alcohol with fluorine. This can effectively inhibit the adhesion of the pollutants such as protein, ureide and the like, so that the measuring device exhibiting stable output characteristics for the operation in longer period can be obtained. In addition, since fluoroalcohol ester group is not dissolved in most of non-fluorine based solvents or in detergent such as the surfactant and the like, the enzyme sensor in having improved chemical resistance is obtained. While the limited permeation layer 140 is composed of a polymer having specific structure, this may also be composed of mixture of two or more of polymers having different structures or molecular weights.

The measurement sample solution, which is the object for the measurement by the biosensor 100 such as the glucose sensor and the like, typically includes blood or urine, or drainage, which often contains various types of foreign materials other than the materials of the object for the measurement. In the cases of such measurement samples, the selective permeation layer 139 or the limited permeation layer 140 are provided in view of preventing the influence, the interference and the interference due to these foreign materials, so that more stable characteristics are maintained to allow exhibiting enhanced quantitativity even in such a severe environment.

### (Fourth Embodiment)

In the above-described embodiment, the calculating unit 105 may alternatively include a correction unit, which corrects the current value when the respective substrate concentrations are acquired by employing I1 and I2.

The correction unit, for example, corrects the current value I2 so as to assume that the concentration of the first substrate is zero at the time T1.

Alternatively, the correction unit may conduct the correction for subtracting the base current value, which is the current value at the time T3 other than T1 and T2 from the respective measured values.

Alternatively, the correction unit may correct the measured value of the concentration of the first or the second substrate on the basis of a predetermined computing equation such as the formula related to Michaelis-Menten kinetics and the like.

Further, the correction unit may conduct a noise elimination processing for the current values measured by the sensor unit 101 as time advances by a predetermined method such as fast Fourier transform, wavelet transformation and the like. This allows enhancing the signal/noise ratio to obtain the concentrations of the respective components in the sample with improved assurance. For example, the method described in ISAO, SHITANDA et al., entitled "Wavelet Transformation of Amperometric Biosensor Responses" BUNSEKIKAGAKU, Vol. 57, No. 3, pp. 183-190 (2008), may also be employed.

While the preferred embodiments of the present invention have been described above in reference to the annexed figures, it should be understood that the disclosures above are presented for the purpose of illustrating the present invention, and various modifications other than that described above are also available.

For example, the above-described embodiments may alternatively be configured that the reaction layer 114 contains an electron acceptor. Such electron acceptor is added to the reaction layer 114 to reduce the influence of dissolved oxygen and interfering substance, so that the quantity of the substrate can be measured by employing a simple structure with an improved accuracy.

It is not necessary to specify the type of the electron acceptor contained in the reaction layer 114, as long as the electron acceptor conducts the exchange of electron with the enzyme. For example, typical electron acceptor may be selected from: metallic complexes and their derivatives such as potassium ferricyanide (potassium ferrocyanide), ferrocene and their derivatives, osmium complex and their derivatives (monomer, polymer), ruthenium complex and the like; quinones; phenazine methosulfate and their derivatives; oxidation-reduction coloring agents such as dichloroindophenol, methylene blue and the like; tetrathiafulvalene (TTF) and their derivatives; and tetracyanoquinodimethane and the like.

Further, the reaction layer 114 may contain a stabilizer. Typical stabilizer includes: saccharides such as trehalose, sucrose, raffinose and the like; amino acids such as arginine, glutamine and the like; polymers; and the like.

Further, the reaction layer 114 can contain a surfactant. The electron acceptor exhibits lower solubility for water. Thus, the electron acceptor may precipitate on the reaction layer due to the presence of water contained in the measurement sample. Therefore, a surfactant is contained in the reaction layer to provide improved solubility for water.

Typical surfactant available in the embodiment includes, for example: lecithin; octyl thioglucoside; sodium cholate; dodecyl-β-maltoside; sodium deoxycholate; sodium taurodeoxycholate; Triton-X 100 (registered trademark); Lubrol PX (registered trademark); DK-ester (registered trademark); BIG CHAP (registered trademark); Deox CHAP (registered trademark); sodium lauryl sulfate (sodium dodecyl sulfate, SDS); sodium dodecylbenzenesulfonate; carboxymethyl cellulose; polyethylene oxide; polyvinyl alcohol; polyvinyl sulfonic acid; poly (diallyldimethylammonium salt); polyacrylic acid; and Tween 20 (registered trademark, polyoxyethylene sorbitan monolaurate). It is particularly preferable to employ Triton-X10 (registered trademark), SDS, and Tween 20 (registered trademark).

Examples of enzymes employed as the first enzyme in the above-described embodiments will be shown below.

### (1.1) Hydrolases Acting on Ester Bonds

These typically include: EC: 3.1.1 carboxylic-ester hydrolases; EC: 3.1.2 trivalent alcohol ester hydrolases (thioester hydrolases); EC: 3.1.3 phosphoric-monoester hydrolases; EC: 3.1.4 phosphoric-diester hydrolases; EC: 3.1.5 triphosphoric-monoester hydrolases; EC: 3.1.6 sulfuric-ester hydrolases; EC: 3.1.7 diphosphoric-monoester hydrolases; EC: 3.1.8 phosphoric-triester hydrolases; EC: 3.1.11 exodeoxyribonucleases producing 5'-phosphomonoesters; EC: 3.1.13 exoribonucleases producing 5'-phosphomonoesters; EC: 3.1.14 exoribonucleases producing 3'-phosphomonoesterse; EC: 3.1.15 exonucleases that are active with either ribo- or deoxyribonucleic acids and produce 5'-phosphomonoesters; EC: 3.1.21 endodeoxyribonucleases producing 5'-phosphomonoesters; EC: 3.1.22 endodeoxyribonucleases producing 3'-phosphomonoesters; EC: 3.1.25 site-specific endodeoxyribonucleases that are specific for altered bases; EC: 3.1.26 endoribonucleases producing 5'-phosphomonoesters; EC: 3.1.27 endoribonucleases producing 3'-phosphomonoesters; EC: 3.1.30 endoribonucleases that are active with either ribo- or deoxyribonucleic acids and produce 5'-phosphomonoesters; and EC: 3.1.31 endoribonucleases that are active with either ribo- or deoxyribonucleic acids and produce 3'-phosphomonoesters, and the like, and preferably the following products are exemplified.

EC: 3.1.1.13: sterol esterase. Hydrolysis of cholesteryl ester is achieved to generate fatty acid and cholesterol. It is measurable by cholesterol oxidase (see Scheme 7). Objective example: blood serum.

EC: 3.1.1.21: retinyl-palmitate esterase. Hydrolysis of retinol ester is carried out into retinol and fatty acid. It is measurable by retinol dehydrogenase. Objective example: blood plasma.

EC: 3.1.1.28: acylcarnitine hydrolase. Hydrolysis of o-acylcarnitine is carried out into L-carnitine and fatty acid. It is measurable by fatty acid dehydrogenase. Objective example: blood plasma, urine.

EC: 3.1.3.25: inositol-phosphate phosphatase. Hydrolysis of 1L-myo-inositol 1-phosphate is carried out into myo- inositol and phosphate. It is measurable by inositol dehydrogenase. Objective example: blood serum.

EC: 3.1.6.4: N-acetylgalactosamine-6-sulfatase. Hydrolysis of N-acetyl-galactosamine-6-sulfate unit (chondroitin sulfate) is carried out into N-acetyl-galactosamine unit (chondroitin sulfate) and sulfate. It is measurable by a combination of EC: 3.2.1.49 and acyl hexosamine oxidase. Object: synovial fluid.

### (1.2) Glycoside Hydrolases (Glycosylases)

These typically include: EC: 3.2.1 glycoside-bond hydrolases (glycosidases) or glycoside hydrolases (glycosylase) and EC: 3.2.2 hydrolysing N-glycosyl compounds and the like, and preferably the following products are exemplified.

EC: 3.2.1.1: α-amylase. Hydrolysis of α-1,4-glucoside bond (bonding status of maltose) of starch (or glycogen) is carried out to provide low molecular weight compound. Oligosaccharide, maltose, and glucose are generated. It is measurable by glucose oxidase or a combination of maltase and glucose oxidase.

EC: 3.2.1.2: β-amylase. Maltose is generated from soluble starch (amylose). It is measurable by a combination of maltase and glucose oxidase.

EC: 3.2.1.4: cellulase. Hydrolysis of β-1,4-glucoside bond (bonding status of lactose (not galactose but glucose)) such as cellulose, lichenin, grain β-glucan and the like is carried out to create β-glucose.

EC: 3.2.1.14: chitinase. β-1,4-glucoside bond in chitin or chitin oligosaccharide is decomposed to generate N-acetylglucosamine or glucosamine. It is measurable by N-acyl hexosamine oxidase.

EC: 3.2.1.20: α-glucosidase. Trivial name: maltase (see Scheme 2).

EC: 3.2.1.21: β-glucosidase. Trivial name: gentiobiase, cellobiase. Hydrolysis of β-D-glucoside (both of gentiobiose (β-1,6-bond) and cellobiose are disaccharide having two glucose bound thereto) is carried out into D-glucose.

EC: 3.2.1.22: α-galactosidase. Trivial name: melibiase. α-1,6-galactoside bond in α-D-galactoside (raffinose) (see Scheme 5, trisaccharide) and stachyose (tetrasaccharide of galactose-galactose-sucrose) are hydrolyzed to generate D-galactose. It is measurable by galactose oxidase. It is also measurable by additionally combining with the decomposition of sucrose.

EC: 3.2.1.23: β-galactosidase. Trivial name: lactase (see Scheme 3).

EC: 3.2.1.24: α-mannosidase. α-D-mannoside is hydrolyzed to generate D-mannose. It is measurable by aldohexose dehydrogenase.

EC: 3.2.1.25: β-mannosidase. β-D-mannoside is hydrolyzed to generate D-mannose. It is measurable by aldohexose dehydrogenase.

EC: 3.2.1.26: β-fructofuranosidase. Trivial name: invertase (see Scheme 1).

EC: 3.2.1.28: a, α-trehalase (see Scheme 4).

EC: 3.2.1.91: cellulose 1,4-β-cellobiosidase. Cellulose or cellotetraose are hydrolyzed to generate cellobiose. It is measurable by a combination of EC: 3.2.1.21.

EC: 3.2.1.108: lactase. Lactose is hydrolyzed to generate D-galactose and D-glucose (see Scheme 3).

### (1.3) Ether Thioether Hydrolases

These typically include: EC: 3.3.1 thioether and trialkylsulfonium hydrolases; and EC: 3.3.2 ether hydrolases.

### (1.4) Hydrolases Acting on Peptide Bonds

These typically include: EC: 3.4.11 amino peptidases; EC: 3.4.13 dipeptidases; EC: 3.4.14 dipeptidyl-peptidases and tripeptidyl-peptidases; EC: 3.4.15 peptidyl-dipeptidases; EC: 3.4.16 serine-type carboxypeptidases; EC: 3.4.17 metallocarboxypeptidases; EC: 3.4.18 cysteine-type carboxypeptidases; EC: 3.4.19 omega peptidases; EC: 3.4.21 serine endopeptidases; EC: 3.4.22 cysteine endopeptidases; EC: 3.4.23 aspartic endopeptidases; EC: 3.4.24 other peptidases (metalloendopeptidases); EC: 3.4.25 threonine endopeptidases, and the like, and preferably the following products are exemplified.

EC: 3.4.11.7: glutamyl aminopeptidase. It is similar to EC: 3.4.11.1. Glutamic acid, and aspartic acid (see Scheme 6).

EC: 3.4.13.7: Glu-Glu dipeptidase. It is similar to EC: 3.4.13.3. Glutamic acid.

EC: 3.4.17.11: glutamate carboxypeptidase. It is similar to EC: 3.4.17.1. Glutamic acid.

EC: 3.4.17.21: glutamate carboxypeptidase II. It is similar to EC: 3.4.17.1. Glutamic acid.

EC: 3.4.19.9: γ-glutamyl hydrolase. It is similar to EC: 3.4.19.1.

EC: 3.4.19.11: γ-D-glutamyl-meso-diaminopimelate peptidase. It is similar to EC: 3.4.19.1.

EC: 3.4.21.82: glutamyl endopeptidase II. It is similar to EC: 3.4.21.1.

### (1.5) Hydrolases Acting on CN bonds Other Than Peptide Bonds

These typically include: EC: 3.5.1 hydrolase acting on linear amides; EC: 3.5.2 hydrolase acting on cyclic amides; EC: 3.5.3 hydrolase acting on linear amidines; EC: 3.5.4 hydrolase acting on cyclic amidines; EC: 3.5.5 hydrolase acting on nitriles; and EC: 3.5.99 hydrolase acting on other compounds, and preferably the following products are exemplified.

EC: 3.5.1.2: glutaminase. L glutamine is hydrolyzed into 1-glutamic acid. It is measurable by glutamate oxidase or the like.

EC: 3.5.1.5: urease. Urea is hydrolyzed into carbon dioxide and ammonia. It is measurable by ammonia mono-oxygenase or the

### like (see Scheme 8).

EC: 3.5.1.35: D-glutaminase. D-glutamine is hydrolyzed into L-glutamic acid and ammonia. It is measurable by glutamate oxidase or ammonia 3-monooxygenase.

EC: 3.5.1.38: glutaminase (asparaginase). L-glutamine (or L-asparagine) is hydrolyzed into L-glutamic acid (or L-asparaginic acid). It is measurable by glutamate oxidase (or amino acid oxidase).

EC: 3.5.1.55: long-chain-fatty-acyl-glutamate deacylase. N-long-chain-fatty-acyl-glutamate is hydrolyzed into long-chain-fatty-acid and L-glutamic acid. It is measurable by glutamate oxidase.

EC: 3.5.1.63: 4-acetamidobutyrate deacetylase. 4-acetamidobutanoate is hydrolyzed into 4-aminobutanoate and acetic acid. It is measurable by a combination of EC: 2.6.1.19: 4-aminobutyrate-transaminase and glutamate oxidase.

EC: 3.5.1.68: N-formylglutamate deformylase. N-formyl-L-glutamate is hydrolyzed into L-glutamic acid and formate. It is measurable by glutamate oxidase or formate dehydrogenase.

EC: 3.5.1.c: β-citryl-glutamate hydrolase. β-citryl-glutamate is hydrolyzed into citric acid and L-glutamic acid. It is measurable by glutamate oxidase.

EC: 3.5.2.9: 5-oxoprolinase. 5-oxo-L-proline is hydrolyzed into L-glutamic acid and adenosinediphosphate (ADP). It is measurable by glutamate oxidase or the like (combination of nuclease + nucleotidase + nucleosidase and the like).

EC: 3.5.2.10: creatininase. Creatinine is hydrolyzed into creatine. It is measurable by EC: 1.5.3.1 sarcosine oxidase. Objective example: urine.

EC: 3.5.3.3: creatinase. Creatine is hydrolyzed into sarcosine and urea. It is measurable by sarcosine oxidase or EC: 3.5.1.5 (see Scheme 9). Objective example: urine.

EC: 3.5.4.21: creatinine deaminase. Creatinine is hydrolyzed into N-methyl hydantoin and ammonia. It is measurable by a combination with EC: 3.5.2.14, or ammonia 3-monooxygenase. Objective example: urine.

### (1.6) Hydrolases Acting on Acid Anhydrides

These typically include: EC: 3.6.1 hydrolases acting on phosphorus-containing anhydrides; EC: 3.6.2 hydrolases acting on sulfonyl-containing anhydrides; EC: 3.6.3 hydrolases acting on acid anhydrides to catalyse transmembrane movement of substances; EC: 3.6.4 hydrolases acting on anhydrides to facilitate cellular and subcellular movement; and EC: 3.6.5 hydrolases acting on GTP to facilitate cellular and subcellular movement.

### (1.7) Hydrolases Acting on Carbon-Carbon bonds

These typically include: EC: 3.7.1 hydrolase acting on ketonic substances.

### (1.8) Hydrolases Acting on Halide Bonds

These typically include: EC: 3.8.1 hydrolase acting on C-halide compounds.

### (1.9) Hydrolases Acting on Phosphorus-Nitrogen Bonds

These typically include: enzyme of EC: 3.9.1 or the like.

### (1.10) Hydrolases acting on Sulfur-Nitrogen Bonds

These typically include: enzyme of EC: 3.10.1 or the like.

### (1.11) Hydrolases Acting on Carbon-Phosphorus Bonds

These typically include: enzyme of EC: 3.11.1 or the like.

### (1.12) Hydrolases Acting on Sulfur-Sulfur Bonds

### (1.13) Hydrolases Acting on Carbon-Sulfur Bonds

These typically include: enzyme of EC: 3.13.1 or the like.

### (2.1) Lyases

These typically include: EC: 4.1.1 lyases acting on carboxy group; EC: 4.1.2 lyases acting on aldehyde group; EC: 4.1.3 oxo-acid-lyase; and EC: 4.1.99 other carbon-carbon lyases and the like.

### (2.2) Carbon-Oxygen Lyases

These typically include: EC: 4.2.1 dehydro-lyases; EC: 4.2.2 lyases acting on polysaccharides; EC: 4.2.3 lyases acting on phosphate group; and EC: 4.2.99 other carbon-oxygen lyases and the like.

### (2.3) C-N Lyases

These typically include: EC: 4.3.1 ammonia-lyases; EC: 4.3.2 amidine-lyases; EC: 4.3.3 amine-lyases and the like.

### (2.4) C-S Lyase

These typically include: EC: 4.4.1 carbon-sulfur lyases, and preferably the following products are exemplified.

EC: 4.4.1.8: cystathionine β-lyase. Cystathionine is converted into L-homocysteine and pyruvic acid and ammonia. It is measurable by various types of enzymes, pyruvate oxidase, and ammonia mono-oxygenase (see Scheme 10). Objective example: blood.

### (2.5) C-Halide Lyases

These typically include: EC: 4.5.1 carbon-halide lyases.

### (2.6) P-O Lyases

These typically include: EC: 4.6.1 phosphorus-oxygen lyases.

### (2.7) Other Lyases

These typically include: EC: 4.99.1 other lyases.

### EXAMPLES

In the present Example, a sensor for measuring sucrose and glucose were produced.

### (Method for Producing Biosensor)

In the present Example, the biosensor having the configuration shown in Fig. 1 was produced. A sensor having a configuration that is similar to the configuration of Fig. 5, in which the sensor unit 101 shown in Fig. 1 was prepared by simplifying the sensor unit 131 shown in Fig. 5 so as not to include the adhesive layer 138 and the selective permeation layer 139, was produced.

A glass base plate 137 provided with a working electrode (platinum electrode) 132, a counter electrode (platinum electrode) 133 and a reference electrode (silver/silver chloride electrode) 135 was prepared.

Further, GOx, invertase, mutarotase and bovine serum albumin (BSA) were dissolved in water, and well mixed and then cooled. Glutaraldehyde was added into this enzyme solution, and after the mixing, the solution is rapidly applied over the electrode through the spin coating process to form the enzyme layer 134. Further, in order to prevent the adsorbing material to the enzyme layer 134, the enzyme layer 134 is coated with a fluororesin to form the limited permeation layer 140, which covers the enzyme layer 134.

### (Electrochemical Measurement)

Standard samples of: glucose solution prepared of 0.1 M TES buffer solution (pH 7.5) containing 150 mM NaCl; sucrose solution prepared of 0.1 M TES buffer solution (pH 7.5) containing 150 mM NaCl; and glucose-sucrose mixed solution prepared of 0.1 M TES buffer solution (pH 7.5) containing 150 mM NaCl were prepared. 5 µL of the sample was added to the reaction layer 114 respectively, and 10 seconds after the addition of the sample, a voltage (+0.3 V/vs) was applied to the working electrode 112 against the counter electrode 113 toward the direction to the anode to commence the measurement of the response current. The current value was measured at 10 seconds to 240 seconds after the commencing of the measurement.

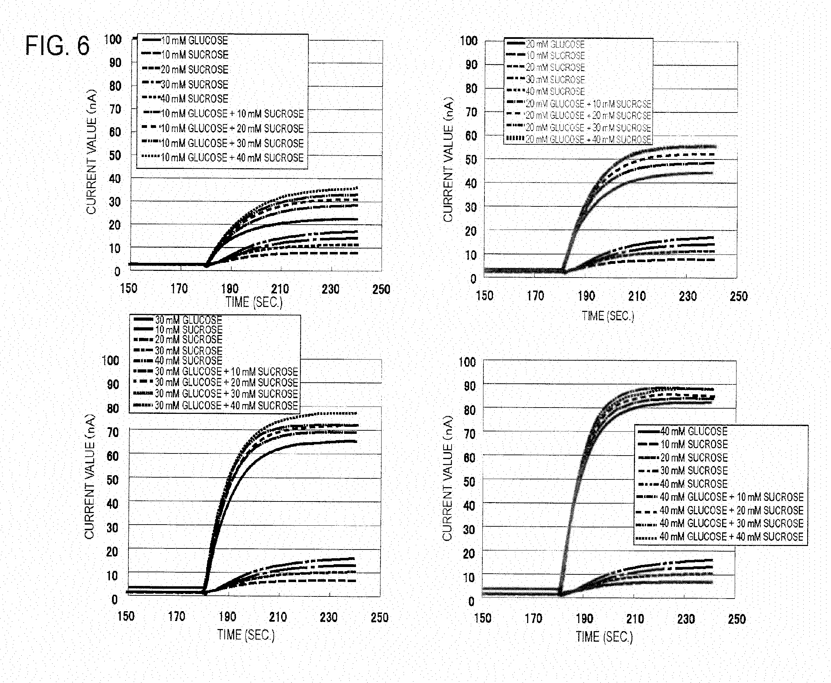

The measurement results obtained by employing the standard samples are shown in Table 1 and Fig. 6. Fig. 6 shows response curves indicating a change of a response current according to a change of the concentration of sucrose or glucose for: the standard sample containing only glucose; the standard sample containing only sucrose; and the standard sample containing glucose and sucrose. Table 1 shows the response current values at the respective time after commencing the measurement, and summarizes the current values at 10 seconds, 20 seconds, 30 seconds, and 60 seconds after the commence of the measurement shown in Fig. 6. Further, in each of the cases of the concentrations of glucose of 0 mM, 10 mM, 20 mM, 30 mM and 40 mM, Table 1 also shows the current values for the concentrations of sucrose of 0 mM, 10 mM, 20 mM, 30 mM and 40 mM.

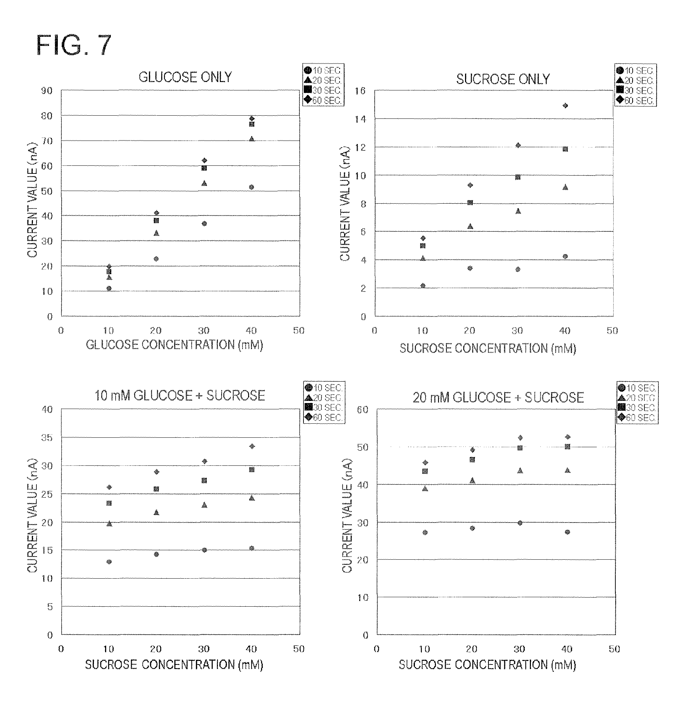

Further, Fig. 7 includes graphs indicating the time-response current value relations obtained by the values of Table 1.

**TABLE-1**

| GLUCOSE ONLY | | | | | SUCROSE ONLY | | | | |
|---|---|---|---|---|---|---|---|---|---|
| GLUCOSE CONCENTRATION | CURRENT VALUE (nA) | | | | SUCROSE CONCENTRATION | CURRENT VALUE (nA) | | | |
| | 10 SEC. AFTER | 20 SEC. AFTER | 30 SEC. AFTER | 60 SEC. AFTER | | 10 SEC. AFTER | 20 SEC. AFTER | 30 SEC. AFTER | 60 SEC. AFTER |
| 10 mM | 11 | 15.6 | 17.8 | 19.8 | 10 mM | 2.2 | 4.1 | 5 | 5.5 |
| 20 mM | 22.7 | 33.2 | 37.9 | 41.1 | 20 mM | 3.4 | 6.4 | 8 | 9.3 |
| 30 mM | 36.7 | 53 | 58.8 | 61.9 | 30 mM | 3.3 | 7.5 | 9.8 | 12.1 |
| 40 mM | 51.4 | 70.8 | 76.4 | 78.7 | 40 mM | 4.2 | 9.2 | 11.9 | 14.9 |

| 10 mM GLUCOSE + SUCROSE | | | | | 20 mM GLUCOSE + SUCROSE | | | | |
|---|---|---|---|---|---|---|---|---|---|
| SUCROSE CONCENTRATION | CURRENT VALUE (nA) | | | | SUCROSE CONCENTRATION | CURRENT VALUE (nA) | | | |
| | 10 SEC. AFTER | 20 SEC. AFTER | 30 SEC. AFTER | 60 SEC. AFTER | | 10 SEC. AFTER | 20 SEC. AFTER | 30 SEC. AFTER | 60 SEC. AFTER |
| 10 mM | 12.9 | 19.8 | 23.3 | 26.2 | 10 mM | 27.2 | 38.9 | 43.4 | 45.8 |
| 20 mM | 14.2 | 21.8 | 25.8 | 28.8 | 20 mM | 28.2 | 41.1 | 43.8 | 43.9 |
| 30 mM | 15 | 23 | 27.3 | 29.2 | 30 mM | 29.7 | 43.8 | 49.6 | 52.4 |
| 40 mM | 15.3 | 24.3 | 29.2 | 33.4 | 40 mM | 27.3 | 43.9 | 50 | 52.6 |

| 30 mM GLUCOSE + SUCROSE | | | | | 40 mM GLUCOSE + SUCROSE | | | | |
|---|---|---|---|---|---|---|---|---|---|
| SUCROSE CONCENTRATION | CURRENT VALUE (nA) | | | | SUCROSE CONCENTRATION | CURRENT VALUE (nA) | | | |
| | 10 SEC. AFTER | 20 SEC. AFTER | 30 SEC. AFTER | 60 SEC. AFTER | | 10 SEC. AFTER | 20 SEC. AFTER | 30 SEC. AFTER | 60 SEC. AFTER |
| 10 mM | 42.5 | 59.7 | 65 | 66.9 | 10 mM | 56.4 | 75 | 79.7 | 81.2 |
| 20 mM | 42.4 | 61 | 67 | 69.6 | 20 mM | 57.8 | 76.7 | 81.2 | 82.2 |
| 30 mM | 44.7 | 63.6 | 68.5 | 69.6 | 30 mM | 60.1 | 79.8 | 84.7 | 84.9 |
| 40 mM | 46.9 | 65 | 71.3 | 74.8 | 40 mM | 58.5 | 78.1 | 82.7 | 85.2 |

According to Fig. 7 and Table 1, calibration curves for each of the standard sample containing only glucose and the standard sample containing only sucrose were obtained within the concentration range of 0 to 40 mM.

Further, concerning the standard sample containing glucose and sucrose, the variation in the current value in accordance with the change of the concentration of sucrose was small at 10 seconds after commencing the measurement, and the response current value derived from the glucose originally contained in the sample was measured. On the other hand, after 20 seconds had passed from the commence of the measurement, the current value was increased and the current value was linearly increased according to the sucrose concentration.

Therefore, the glucose concentration in the standard sample containing glucose and sucrose can be acquired by utilizing the calibration curve prepared by the standard sample containing only glucose from, for example, the current value obtained at 10 seconds after commencing the measurement. Also, the sucrose concentration in the standard sample containing glucose and sucrose can also be acquired by utilizing the calibration curve prepared by the standard sample containing only sucrose from the current value obtained after 20 seconds had passed from the commence of the measurement, or for example, the current value obtained at 30 seconds after commencing the measurement.

It is apparent that the present invention is not limited to the above embodiment, and may be modified and changed without departing from the scope and spirit of the invention.

## Claims

1. A biosensor for measuring concentrations of first and second substrates in a sample, comprising:
a sensor unit including a base plate, an electrode provided over said base plate, and a reaction layer provided over said electrode;
a timing unit for measuring time; and
a calculating unit,
wherein said reaction layer comprises a first enzyme for converting said first substrate into said second substrate, and a second enzyme acting on said second substrate or a converted substance obtainable by further converting said second substrate, and
wherein said calculating unit calculates a concentration of said first substrate and a concentration of said second substrate in said sample respectively, on the basis of a first current value detected in said sensor unit at a first time and a second current value detected in said sensor unit at a second time.

2. The biosensor according to claim 1, wherein said electrode has a working electrode, and said reaction layer is provided over said working electrode.

3. The biosensor according to claim 1 or 2, wherein said calculating unit calculates the concentration of said second substrate in said sample on the basis of said first current value, and calculates the concentration of said first substrate in said sample on the basis of a difference between said second current value and said first current value.

4. The biosensor according to any one of claims 1 to 3, wherein said second enzyme is an oxidoreductase.

5. The biosensor according to any one of claims 1 to 4, wherein said first enzyme is hydrolase of said first substrate.

6. The biosensor according to any one of claims 1 to 5, wherein said first substrate is one selected from the group consisting of sucrose, maltose, lactose and trehalose,
wherein said second substrate is glucose, and
wherein said second enzyme is a glucose oxidase or a glucose reductase.

7. The biosensor according to any one of claims 1 to 5, wherein said second substrate is glutamic acid, and said second enzyme is a glutamate oxidase.

8. The biosensor according to any one of claims 1 to 5, wherein said first substrate is one selected from the group consisting of cholesteryl ester, urea, creatinine, cystathionine and raffinose.

9. The biosensor according to any one of claims 1 to 8, wherein said reaction layer includes a first reaction layer containing said first enzyme and a second reaction layer containing said second enzyme.

10. A method of measuring said first and said second substrate concentrations in a sample by employing the biosensor according to any one of claims 1 to 9, including:
acquiring said first current value detected at said first time in said sensor unit;
acquiring said second current value detected at said second time in said sensor unit; and
calculating a concentration of said first substrate and a concentration of said second substrate in said sample respectively, on the basis of said first and said second current values.
